(19) Europäisches Patentamt
European Patent Office
Office européen des brevets

(11) **EP 4 360 753 A1**

(12) **EUROPEAN PATENT APPLICATION**
published in accordance with Art. 153(4) EPC

(43) Date of publication:
**01.05.2024 Bulletin 2024/18**

(21) Application number: **22828320.6**

(22) Date of filing: **16.06.2022**

(51) International Patent Classification (IPC):
**B01J 23/10** (2006.01)  **B01J 23/02** (2006.01)
**B01J 23/08** (2006.01)  **B01J 23/78** (2006.01)
**C07B 61/00** (2006.01)  **C07C 2/84** (2006.01)
**C07C 11/04** (2006.01)

(52) Cooperative Patent Classification (CPC):
**B01J 23/02; B01J 23/08; B01J 23/10; B01J 23/78;
C07B 61/00; C07C 2/84; C07C 11/04**

(86) International application number:
**PCT/JP2022/024126**

(87) International publication number:
**WO 2022/270403 (29.12.2022 Gazette 2022/52)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **25.06.2021 JP 2021105825**

(71) Applicant: **Niterra Co., Ltd.
Nagoya-shi, Aichi 461-0005 (JP)**

(72) Inventors:
• **WATANABE, Shuntaro
Nagoya-shi, Aichi 461-0005 (JP)**
• **NAKAMURA, Yosuke
Nagoya-shi, Aichi 461-0005 (JP)**
• **KOZUKA, Hisashi
Nagoya-shi, Aichi 461-0005 (JP)**

(74) Representative: **Grünecker Patent- und
Rechtsanwälte
PartG mbB
Leopoldstraße 4
80802 München (DE)**

(54) **DEHYDROGENATION REACTION CATALYST, COMPOSITE CATALYST AND SUPPORTED CATALYST**

(57)   A dehydrogenation reaction catalyst has a perovskite structure represented by the general formula $(A_{1-x}A'_x)(Zr_{1-y-z}B_yB'_z)O_3$ (where A is at least one element selected from alkaline earth metals, A' is at least one of lanthanum (La) and yttrium (Y), B is at least one of titanium (Ti) and cerium (Ce), and B' is at least one element selected from yttrium (Y), scandium (Sc), ytterbium (Yb), aluminum (Al), indium (In), and neodymium (Nd)). x, Y, and z satisfy $0 \le x \le 0.4$, $0.3 \le (1 - z) \le 1$, $0 \le y$, and $0 < (1 - y - z)$.

FIG. 5

**Description**

Technical Field

The present disclosure relates to dehydrogenation reaction catalysts, composite catalysts, and supported catalysts.

Background Art

Conventionally known methods for producing ethylene include a method involving thermal cracking and fractionation of higher hydrocarbons such as naphtha and a method involving thermal cracking of ethane derived from natural gas. Another method that has been proposed is a method for producing ethylene by converting methane into ethylene through a methane oxidative coupling reaction using a catalyst (see, for example, NPL 1).

Citation List

Non Patent Literature

NPL 1: Brittany Lancaster Farrell et al., ACS Catal. 2016, 6, 4340-4346

Summary of Invention

Technical Problem

A method using a methane oxidative coupling catalyst is advantageous over a method for producing ethylene by thermal cracking of hydrocarbons in that the amount of energy supplied for ethylene production can be reduced. However, conventionally known methane oxidative coupling catalysts do not necessarily have sufficient catalytic activity, and a technique for increasing the catalytic activity of a methane oxidative coupling catalyst has been desired. Furthermore, a technique for increasing the activity of a catalyst that promotes a reaction, other than a methane oxidative coupling reaction, involving a process of dehydrogenating a reactant as in a methane oxidative coupling reaction has been similarly desired.

Solution to Problem

The present disclosure can be implemented in the following aspects:

(1) An aspect of the present disclosure provides a dehydrogenation reaction catalyst. This dehydrogenation reaction catalyst has a perovskite structure represented by the general formula $(A_{1-x}A'_{x})(Zr_{1-y-z}B_{y}B'_{z})O_{3}$ (where A is at least one element selected from alkaline earth metals, A' is at least one of lanthanum (La) and yttrium (Y), B is at least one of titanium (Ti) and cerium (Ce), and B' is at least one element selected from yttrium (Y), scandium (Sc), ytterbium (Yb), aluminum (Al), indium (In), and neodymium (Nd)). x, y, and z satisfy:

$$0 \leq x \leq 0.4,$$

$$0.3 \leq (1 - z) \leq 1,$$

$$0 \leq y,$$

and

$$0 < (1 - y - z).$$

The dehydrogenation reaction catalyst in accordance with this aspect can exhibit an increased activity of promoting a dehydrogenation reaction.

(2) The dehydrogenation reaction catalyst in accordance with the above aspect may be an alkane oxidative coupling catalyst that promotes an alkane oxidative coupling reaction that couples a reactant including at least one of an alkane and an alkane derivative. With this configuration, the activity of promoting an alkane oxidative coupling reaction can be increased.

(3) The dehydrogenation reaction catalyst in accordance with the above aspect may be a methane oxidative coupling catalyst. With this configuration, the activity of promoting a methane oxidative coupling reaction can be increased.

(4) The dehydrogenation reaction catalyst in accordance with the above aspect may have a total conductivity of $1.0 \times 10^{-5}$ S/cm or more and $1.0 \times 10^{-1}$ S/cm or less. With this configuration, since the dehydrogenation reaction catalyst, serving as a methane oxidative coupling catalyst, has a total conductivity of $1.0 \times 10^{-5}$ S/cm or more, the activity of promoting a methane oxidative coupling reaction can be further increased. In addition, since the methane oxidative coupling catalyst has a total conductivity of $1.0 \times 10^{-1}$ S/cm or less, excessive oxidation of methane, that is, a complete oxidation reaction that produces carbon dioxide and water from methane, can be inhibited, and accordingly, the activity of promoting a methane oxidative coupling reaction can be increased.

(5) The dehydrogenation reaction catalyst in accordance with the above aspect may have a proton conductivity of $1.0 \times 10^{-4}$ S/cm or more. With this configuration, the activity of promoting a dehydrogenation reaction can be further increased.

(6) The dehydrogenation reaction catalyst in accordance with the above aspect may have a proton transport number of 0.01 or more. With this configuration, the activity of promoting a dehydrogenation reaction can be further increased.

(7) In the dehydrogenation reaction catalyst in accordance with the above aspect, x may satisfy:

$$0 \leq x \leq 0.2.$$

With this configuration, the activity of promoting a dehydrogenation reaction can be further increased.

(8) The dehydrogenation reaction catalyst in accordance with the above aspect may have a sum of an electron transport number and a hole transport number of 0.01 or more and 0.95 or less. With this configuration, since the dehydrogenation reaction catalyst has a sum of the electron transport number and the hole transport number of 0.01 or more, the activity of promoting a dehydrogenation reaction that is a methane oxidative coupling reaction can be further increased. In addition, since the dehydrogenation reaction catalyst has a sum of the electron transport number and the hole transport number of 0.95 or less, excessive oxidation of methane, that is, a complete oxidation reaction that produces carbon dioxide and water from methane, can be inhibited, and accordingly, the activity of promoting a methane oxidative coupling reaction can be increased. (9) The dehydrogenation reaction catalyst in accordance with the above aspect may have a proton transport number of 0.10 or more and a sum of an electron transport number and a hole transport number of 0.10 or more. With this configuration, the activity of promoting a dehydrogenation reaction can be further increased.

(10) The dehydrogenation reaction catalyst in accordance with the above aspect may have a perovskite structure represented by the general formula $BaZr_{1-z}B'_zO_3$ (where B' is at least one element selected from yttrium (Y), scandium (Sc), ytterbium (Yb), indium (In), and neodymium (Nd)). With this configuration, the activity of promoting a dehydrogenation reaction can be further increased.

(11) Another aspect of the present disclosure provides a composite catalyst. This composite catalyst includes the methane oxidative coupling catalyst in accordance with (3) or (4) and at least one of a metal catalyst and an oxide catalyst.

The composite catalyst in accordance with this aspect can exhibit a further increased function of promoting a methane oxidative coupling reaction.

(12) Still another aspect of the present disclosure provides a supported catalyst including a catalytic component supported on a support. In this supported catalyst, the catalytic component includes the methane oxidative coupling catalyst in accordance with (3) or (4).

The supported catalyst in accordance with this aspect allows a methane oxidative coupling catalyst having an increased activity of promoting a methane oxidative coupling reaction to be supported and used on a support.

(13) Still another aspect of the present disclosure provides a supported catalyst including a catalytic component supported on a support. In this supported catalyst, the catalytic component includes the composite catalyst in accordance with (11).

The supported catalyst in accordance with this aspect allows a composite catalyst having an increased activity of promoting a methane oxidative coupling reaction to be supported and used on a support.

The present disclosure can be implemented in various aspects other than those described above. For example, the present disclosure can be implemented in aspects such as a method for producing a dehydrogenation reaction catalyst, a method for producing a methane oxidative coupling catalyst, and an ethylene production apparatus including a methane oxidative coupling catalyst.

Brief Description of Drawings

[Fig. 1] Fig. 1 is an explanatory diagram schematically showing an outline of a methane oxidative coupling reaction.
[Fig. 2] Fig. 2 is an explanatory diagram showing, in detail, an example reaction that proceeds over a methane oxidative coupling catalyst.
[Fig. 3] Fig. 3 is an explanatory diagram showing, in detail, an example reaction that proceeds over the methane oxidative coupling catalyst.
[Fig. 4] Fig. 4 is an explanatory diagram showing various dehydrogenation reactions that can be promoted by a dehydrogenation reaction catalyst.
[Fig. 5] Fig. 5 is an explanatory diagram showing a schematic configuration of an ethylene production apparatus.
[Fig. 6] Fig. 6 is an explanatory diagram showing test results for the performance of methane oxidative coupling catalysts.
[Fig. 7] Fig. 7 is an explanatory diagram showing test results for the performance of methane oxidative coupling catalysts.

Description of Embodiments

A. Dehydrogenation Reaction Catalyst:

A dehydrogenation reaction catalyst in accordance with the present embodiment is a composite oxide including a crystal phase having a perovskite-type oxide crystal structure satisfying the following general formula, namely, formula (1). The dehydrogenation reaction catalyst in accordance with the present embodiment promotes various reactions involving a process of dehydrogenating a reactant or the like. An example of a reaction promoted by the dehydrogenation reaction catalyst is a methane oxidative coupling reaction. In the present specification, an overall reaction involving a process of dehydrogenating a reactant or the like, such as a methane oxidative coupling reaction, is also referred to as "dehydrogenation reaction". The dehydrogenation reaction catalyst in accordance with the present embodiment can be a methane oxidative coupling catalyst. A methane oxidative coupling catalyst is a catalyst that promotes a methane oxidative coupling (oxidative coupling of methane: OCM) reaction that converts methane into a $C_2$ hydrocarbon such as ethylene. In addition to the activity of promoting a dehydrogenation reaction, the dehydrogenation reaction catalyst in accordance with the present embodiment has proton conductivity and at least one of electron conductivity and hole conductivity. The dehydrogenation reaction promoted by the dehydrogenation reaction catalyst in accordance with the present embodiment, such as a methane oxidative coupling reaction, will be described in detail later.

$$(A_{1-x}A'_x)(Zr_{1-y-z}B_yB'_z)O_3 \qquad (1)$$

where A is at least one element selected from alkaline earth metals, A' is at least one of lanthanum (La) and yttrium (Y), B is at least one of titanium (Ti) and cerium (Ce), and B' is at least one element selected from yttrium (Y), scandium (Sc), ytterbium (Yb), aluminum (Al), indium (In), and neodymium (Nd). In addition, x, Y, and z in formula (1) above satisfy the following relationships:

$$0 \le x \le 0.4 \qquad (2a)$$

$$0.3 \le (1 - z) \le 1 \qquad (2b)$$

$$0 \le y \qquad (2c)$$

$$0 < (1 - y - z) \qquad (2d)$$

As shown in formula (1) above, the dehydrogenation reaction catalyst in accordance with the present embodiment contains at least one element selected from alkaline earth metals in the so-called A sites of the perovskite structure. It is desirable that the alkaline earth metal be an element selected from barium (Ba), calcium (Ca), and strontium (Sr). The alkaline earth metal element present in the A sites is hereinafter also referred to as "element A". The dehydrogenation reaction catalyst in accordance with the present embodiment may further contain at least one of lanthanum (La) and yttrium (Y) in the A sites of the perovskite structure. However, lanthanum (La) and yttrium (Y) are not essential. Lanthanum (La) and yttrium (Y) present in the A sites are also hereinafter referred to as "element A'". The amounts of the element A and the element A' in the dehydrogenation reaction catalyst in accordance with the present embodiment satisfy inequality (2a) in formula (1) described above.

In addition, the dehydrogenation reaction catalyst in accordance with the present embodiment contains zirconium (Zr) in the so-called B sites of the perovskite structure. The dehydrogenation reaction catalyst in accordance with the present embodiment may further contain at least one of titanium (Ti) and cerium (Ce) in the B sites of the perovskite structure. However, titanium (Ti) and cerium (Ce) are not essential. Titanium (Ti) and cerium (Ce) present in the B sites are hereinafter also referred to as "element B". The dehydrogenation reaction catalyst in accordance with the present embodiment may further contain at least one element selected from yttrium (Y), scandium (Sc), ytterbium (Yb), aluminum (Al), indium (In), and neodymium (Nd) in the B sites of the perovskite structure. However, at least one element selected from yttrium (Y), scandium (Sc), ytterbium (Yb), aluminum (Al), indium (In), and neodymium (Nd) is not essential. At least one element selected from yttrium (Y), scandium (Sc), ytterbium (Yb), aluminum (Al), indium (In), and neodymium (Nd) present in the B sites is hereinafter also referred to as "element B'". The amounts of Zr, the element B, and the element B' in the dehydrogenation reaction catalyst in accordance with the present embodiment satisfy inequalities (2b) to (2d) in formula (1) described above.

The thus-configured dehydrogenation reaction catalyst in accordance with the present embodiment can exhibit an increased activity (hereinafter also referred to as "catalytic activity") of promoting a dehydrogenation reaction (e.g., a methane oxidative coupling reaction that converts methane into a $C_2$ hydrocarbon such as ethylene). Because of the increased activity of promoting a dehydrogenation reaction, it is believed that, for example, when the dehydrogenation reaction catalyst in accordance with the present embodiment is used as a methane oxidative coupling catalyst, the catalyst can exhibit an increased activity of promoting a dehydrogenation reaction of methane, serving as a reactant, that is, a reaction that produces methyl radicals from methane, thus promoting the overall methane oxidative coupling reaction. Because of the increased catalytic activity, for example, the dehydrogenation reaction catalyst in accordance with the present embodiment can be used as a methane oxidative coupling catalyst to increase the ethylene production efficiency. The dehydrogenation reaction, such as a reaction that produces methyl radicals from methane, will be described in detail later.

For the dehydrogenation reaction catalyst in accordance with the present embodiment, it is desirable that x in formula (1) described above satisfy inequality (2e) below. This allows the activity of promoting a dehydrogenation reaction to be further increased.

$$0 \le x \le 0.2 \quad (2e)$$

In addition, it is desirable that the dehydrogenation reaction catalyst in accordance with the present embodiment have a perovskite structure represented by the following general formula, namely, formula (3). This allows the activity of promoting a dehydrogenation reaction to be further increased.

$$BaZr_{1-z}B'_zO_3 \quad (3)$$

where B' is at least one element selected from yttrium (Y), scandium (Sc), ytterbium (Yb), indium (In), and neodymium (Nd).

In addition, the dehydrogenation reaction catalyst in accordance with the present embodiment may be used to form a composite catalyst including the dehydrogenation reaction catalyst and at least one of a metal catalyst and an oxide catalyst. If the dehydrogenation reaction catalyst in accordance with the present embodiment and a metal catalyst or an oxide catalyst are combined into a composite catalyst, for example, by mixing them together, the composite catalyst is more effective in promoting the overall desired dehydrogenation reaction. Metal catalysts and oxide catalysts are known to promote an oxidation reaction as well as a dehydrogenation reaction. Therefore, if an oxidation reaction proceeds due to the use of a metal catalyst or an oxide catalyst, carbon dioxide can be produced as a product instead of the intended product such as ethylene. However, when a metal catalyst or an oxide catalyst is combined with the dehydrogenation reaction catalyst as in the present embodiment, a dehydrogenation reaction proceeds preferentially over an oxidation reaction, thus further promoting the desired reaction such as ethylene production. The metal catalyst or oxide catalyst combined with the dehydrogenation reaction catalyst may be selected as appropriate depending on the desired dehydrogenation reaction. As the metal catalyst, for example, a metal selected from palladium (Pd), copper (Cu), iron (Fe), nickel (Ni), platinum (Pt), indium (In), manganese (Mn), ruthenium (Ru), strontium (Sr), zinc (Zn), and lithium (Li) can be used. As the oxide catalyst, for example, an oxide selected from $Li_2ASiO_4$ (where A is Ca or Sr), $Ce_2(WO_4)_3$, and $CeO_2$ can be used.

B. Methane Oxidative Coupling Reaction:

A methane oxidative coupling reaction promoted by a methane oxidative coupling catalyst when the dehydrogenation reaction catalyst in accordance with the present embodiment is used as the methane oxidative coupling catalyst will be described below.

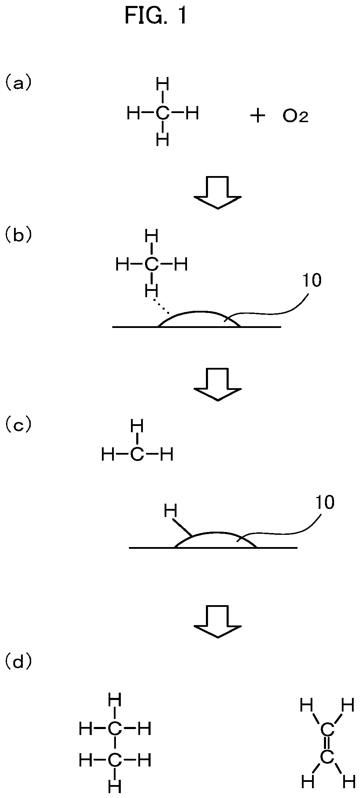

Fig. 1 is an explanatory diagram schematically showing an outline of the methane oxidative coupling reaction. The production of $C_2$ hydrocarbons by the methane oxidative coupling reaction is performed, for example, in the presence of oxygen. As shown in Fig. 1, when the methane oxidative coupling reaction proceeds, a reaction that produces methyl radicals from methane (methane dehydrogenation reaction) proceeds over a methane oxidative coupling catalyst 10. $C_2$ hydrocarbons such as ethylene and ethane are then produced from the resulting methyl radicals. Here, the covalent bonds between the carbon and hydrogen atoms of methane are extremely stable bonds with a binding energy of 104 kcal/mol. Because methane has stable covalent bonds as described above, the reaction that produces methyl radicals from methane (the reaction from (b) to (c) in Fig. 1) is generally thought to be a rate-limiting step of the methane oxidative coupling reaction. To promote the overall methane oxidative coupling reaction, therefore, it is important to promote the above-described reaction that produces methyl radicals from methane. Because the methane oxidative coupling catalyst 10 in accordance with the present embodiment has a high activity of promoting the above-described reaction that produces methyl radicals from methane, it is believed that the methane oxidative coupling catalyst 10 has a high activity of promoting the methane oxidative coupling reaction and can increase the ethylene production efficiency.

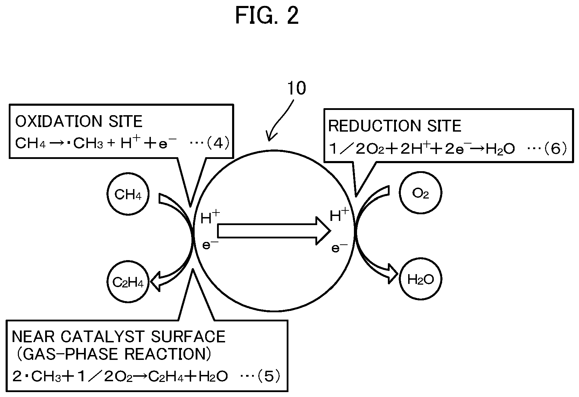

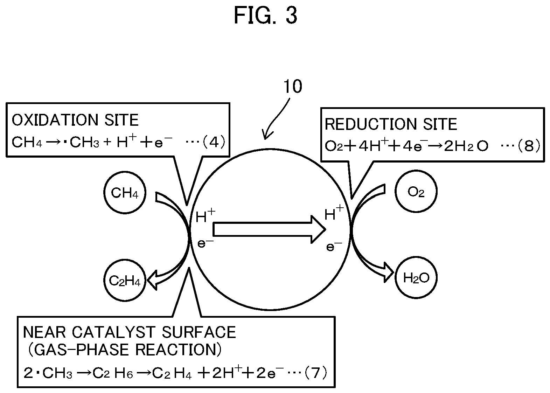

Figs. 2 and 3 are explanatory diagrams showing, in greater detail, example methane oxidative coupling reactions that proceed over the methane oxidative coupling catalyst 10 in accordance with the present embodiment. Figs. 2 and 3 show the methane oxidative coupling catalyst 10 as being formed in powder or granular form. As shown in Figs. 2 and 3, when a methane oxidative coupling reaction proceeds, a reaction that produces methyl radicals from methane proceeds first, as in formula (4) below.

$$CH_4 \rightarrow \cdot CH_3 + H^+ + e^- \qquad (4)$$

Subsequently, it is thought that reactions of formulas (5) and (6) shown below (see Fig. 2) or reactions of formulas (7) and (8) shown below (see Fig. 3) proceed over the methane oxidative coupling catalyst, thus producing substances such as ethylene.

$$2\cdot CH_3 + 1/2O_2 \rightarrow C_2H_4 + H_2O \qquad (5)$$

$$1/2O_2 + 2H^+ + 2e^- \rightarrow H_2O \qquad (6)$$

$$2 \cdot CH_3 \rightarrow C_2H_6 \rightarrow C_2H_4 + 2H^+ + 2e^- \qquad (7)$$

$$O_2 + 4H^+ + 4e^- \rightarrow 2H_2O \qquad (8)$$

That is, it is thought that, as the reactions of formulas (4) to (6) shown in Fig. 2 or the reactions of formulas (4), (7), and (8) shown in Fig. 3 proceed over the methane oxidative coupling catalyst, a reaction of formula (9) shown below proceeds as a whole.

$$2CH_4 + O_2 \rightarrow C_2H_4 + 2H_2O \qquad (9)$$

In Figs. 2 and 3, the site where the reaction of formula (4) that produces methyl radicals from methane proceeds is indicated as "oxidation site". Figs. 2 and 3 show that the reaction of formula (5) or (7) that further produces ethylene from methyl radicals proceeds at the oxidation site. In addition, in Figs. 2 and 3, the site where the reaction of formula (6) or (8) that produces water from protons and electrons produced together with methyl radicals in formula (4) is indicated as "reduction site". These reactions can proceed at any site over the methane oxidative coupling catalyst. For the reaction of formula (9) that produces a $C_2$ hydrocarbon such as ethylene from methane to proceed sufficiently, it is desirable that, in addition to a high activity of promoting the reaction of formula (4), which is the rate-limiting step described above, the methane oxidative coupling catalyst have high proton conductivity and electron conductivity (including hole conductivity) so that, after the reaction of formula (4), the subsequent reactions such as the reactions of formulas (5) and (6) or the reactions of formulas (7) and (8) proceed smoothly. Specifically, it is desirable that the methane oxidative coupling catalyst have a higher proton conductivity or proton transport number or a higher electron conductivity (including hole conductivity) or electron transport number (including hole transport number). In addition, it is desirable that the methane oxidative coupling catalyst have a higher total conductivity, that is, a higher sum of the proton conductivity and the electron conductivity (including hole conductivity), as a whole.

It is desirable that the methane oxidative coupling catalyst have a total conductivity of $1.0 \times 10^{-5}$ S/cm or more and $1.0 \times 10^{-1}$ S/cm or less. A total conductivity of $1.0 \times 10^{-5}$ S/cm or more and $1.0 \times 10^{-1}$ S/cm or less can be easily achieved when the methane oxidative coupling catalyst in accordance with the present embodiment satisfies formula (1) and inequalities (2a) to (2d) described above. If the methane oxidative coupling catalyst has a total conductivity of $1.0 \times 10^{-5}$ S/cm or more, the catalytic activity can be further increased. If the methane oxidative coupling catalyst has a total conductivity of more than $1.0 \times 10^{-1}$ S/cm, excessive oxidation of methane, that is, a complete oxidation reaction that produces carbon dioxide and water from methane, generally proceeds easily due to excessively high electron conductivity (including hole conductivity). Thus, if the methane oxidative coupling catalyst has a total conductivity of $1.0 \times 10^{-1}$ S/cm or less, the above-described complete oxidation reaction of methane can be inhibited, and accordingly, the catalytic activity of producing ethylene from methane can be increased.

In addition, it is desirable that the methane oxidative coupling catalyst have a proton conductivity of $1.0 \times 10^{-4}$ S/cm or more. A proton conductivity of $1.0 \times 10^{-4}$ S/cm or more can be easily achieved when the methane oxidative coupling catalyst in accordance with the present embodiment satisfies formula (1) and inequalities (2a) to (2d) described above. If the methane oxidative coupling catalyst has a proton conductivity within the above range, the catalytic activity can be further increased. Although there is no particular upper limit on the proton conductivity of the methane oxidative coupling catalyst, the methane oxidative coupling catalyst typically has a proton conductivity of $1.0 \times 10^{-1}$ S/cm or less.

In addition, it is desirable that the methane oxidative coupling catalyst have a proton transport number of 0.01 or more. A proton transport number of 0.01 or more can be easily achieved when the methane oxidative coupling catalyst in accordance with the present embodiment satisfies formula (1) and inequalities (2a) to (2d) described above. If the methane oxidative coupling catalyst has a proton transport number within the above range, the catalytic activity can be further increased. Although there is no particular upper limit on the proton transport number of the methane oxidative coupling catalyst, it is desirable that the methane oxidative coupling catalyst have a proton transport number of 0.99 or less, more desirably 0.90 or less, from the viewpoint of ensuring the electron transport number (including hole transport number).

In addition, it is desirable that the methane oxidative coupling catalyst have a sum of the electron transport number and the hole transport number of 0.01 or more and 0.95 or less. A sum of the electron transport number and the hole transport number of 0.01 or more and 0.95 or less can be easily achieved when the methane oxidative coupling catalyst in accordance with the present embodiment satisfies formula (1) and inequalities (2a) to (2d) described above. If the methane oxidative coupling catalyst has a sum of the electron transport number and the hole transport number of 0.01 or more, the catalytic activity can be further increased. If the methane oxidative coupling catalyst has a sum of the electron transport number and the hole transport number of 0.95 or less, excessive oxidation of methane, that is, a complete oxidation reaction that produces carbon dioxide and water from methane, can be inhibited, and accordingly, the catalytic activity of producing ethylene from methane can be increased.

Furthermore, it is desirable that the methane oxidative coupling catalyst have a proton transport number of 0.10 or more and a sum of the electron transport number and the hole transport number of 0.10 or more. A proton transport number of 0.10 or more and a sum of the electron transport number and the hole transport number of 0.10 or more can be easily achieved when the methane oxidative coupling catalyst in accordance with the present embodiment satisfies formula (1) and inequalities (2a) to (2d) described above. If the methane oxidative coupling catalyst has a proton transport number of 0.10 or more and a sum of the electron transport number and the hole transport number of 0.10 or more, the catalytic activity can be further increased.

C. Other Dehydrogenation Reactions:

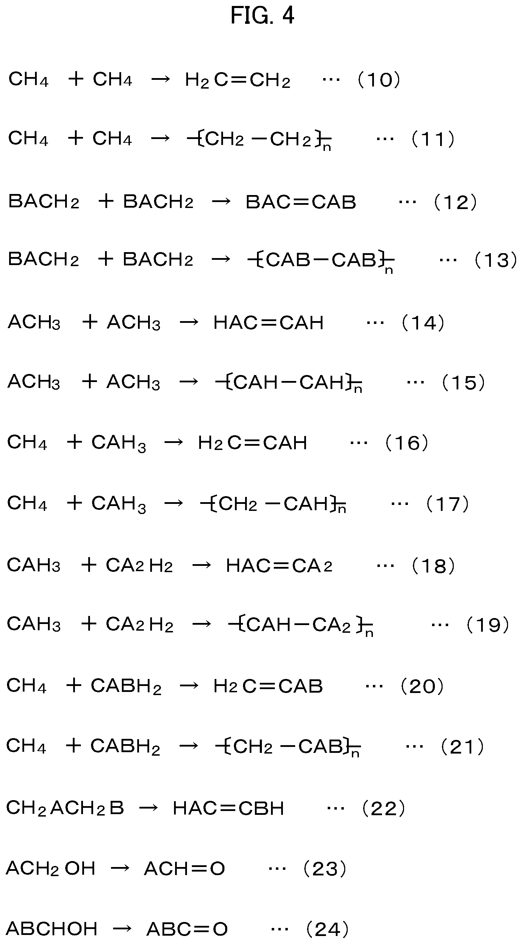

Fig. 4 is an explanatory diagram showing various examples of dehydrogenation reactions that can be promoted by the dehydrogenation reaction catalyst in accordance with the present embodiment. The dehydrogenation reaction catalyst in accordance with the present embodiment can promote various reactions depending on the raw material (reactant) used. Formulas (10) to (21) in Fig. 4 show reactions that couple a reactant including at least one of methane and a methane derivative (hereinafter also referred to as "methane"). Such reactions are also referred to as "methane oxidative coupling reaction". A dehydrogenation reaction catalyst in accordance with the present embodiment that promotes a methane oxidative coupling reaction is also referred to as "methane oxidative coupling catalyst". In formulas (12) to (21) in Fig. 4, A and B in the methane derivatives each represent an atom or atomic group serving as a substituent with which a hydrogen atom in the methane molecule has been replaced, and n is an integer of 2 or more. The methane derivatives shown in Fig. 4 have one or two hydrogen atoms in the methane molecule replaced with the above substituents.

If one molecule has the substituent A and the substituent B, A and B may be the same substituent or different substituents. Examples of substituents represented by A or B include halogen elements, hydroxy groups (-OH), and phenyl groups ($-C_6H_5$).

In Fig. 4, formula (10) represents a reaction that produces ethylene from methane, as with formula (9). Formula (11) shows a reaction that further produces polyethylene as a polymerization reaction proceeds. Formulas (10) to (21) show only changes in, of the molecules involved in the reactions, methane and the methane derivatives, unlike formula (9).

Formulas (10) and (11) show reactions in which the reactant is methane. Formulas (12) and (13) show reactions in which the reactant is a methane derivative represented as $BACH_2$. Formulas (14) and (15) show reactions in which the reactant is a methane derivative represented as $ACH_3$. Formulas (16) and (17) show reactions in which the reactants are methane and a methane derivative represented as $ACH_3$. Formulas (18) and (19) show reactions in which the reactants are a methane derivative represented as $ACH_3$ and a methane derivative represented as $CA_2H_2$. Formulas (20) and (21) show reactions in which the reactants are methane and a methane derivative represented as $CABH_2$.

In addition, formulas (10), (12), (14), (16), (18), and (20) show reactions that produce a hydrocarbon having two carbon atoms (ethylene) or an ethylene derivative (hereinafter also referred to as "$C_2$ hydrocarbon") using a reactant selected from a hydrocarbon having one carbon atom (methane) and a methane derivative (hereinafter also referred to as "$C_1$ hydrocarbon"). Formulas (11), (13), (15), (17), (19), and (21) show reactions that produce a polymer using a reactant selected from $C_1$ hydrocarbons. When a polymer is produced, a $C_2$ hydrocarbon may be produced from a $C_1$ hydrocarbon and may then be polymerized with itself. Alternatively, a $C_1$ hydrocarbon may be sequentially polymerized at the ends of the molecule to form a polymer. Both of these reactions may also proceed.

The reactions shown in Fig. 4 will be more specifically described. For example, in formula (13), if the substituents A and B are both fluorine atoms (F), the resulting product is polytetrafluoroethylene (PTFE). In formula (17), if the substituent A is a chlorine atom (Cl), the resulting product is polyvinyl chloride (PVC), if the substituent A is a hydroxy group (-OH), the resulting product is polyvinyl alcohol (PVOH), and if the substituent A is a phenyl group ($-C_6H_5$), the resulting product is polystyrene (PS). In formula (21), if the substituents A and B are both fluorine atoms (F), the resulting product is polyvinylidene fluoride (PVDF), and if the substituents A and B are both chlorine atoms (Cl), the resulting product is polyvinylidene chloride (PVDC). In addition, in formula (21), if the substituent A is a methyl group ($-CH_3$) and the substituent B is a methoxycarbonyl group ($-COOCH_3$), the resulting product is polymethyl methacrylate (PMMA).

Although Fig. 4 shows methane oxidative coupling reactions, the dehydrogenation reaction catalyst in accordance with the present embodiment can also be used to promote dehydrogenation reactions other than methane oxidative coupling reactions. Examples of dehydrogenation reactions promoted by the dehydrogenation reaction catalyst in accordance with the present embodiment include reactions that couple a reactant including at least one of an alkane and an alkane derivative (hereinafter also referred to as "alkane"). Such reactions, including the methane oxidative coupling reactions described above, are also referred to as "alkane oxidative coupling reaction". A dehydrogenation reaction catalyst in accordance with the present embodiment that promotes an alkane oxidative coupling reaction is also referred to as "alkane oxidative coupling catalyst". The alkane used as the reactant in the alkane oxidative coupling reaction promoted by the dehydrogenation reaction catalyst in accordance with the present embodiment may have, for example, 1 to 10 carbon atoms, preferably 1 to 5 carbon atoms, more preferably 1 or 2 carbon atoms. The alkane derivative used in the alkane oxidative coupling reaction, which has a hydrogen atom in the alkane molecule replaced with a substituent as described above, have a covalent bond between carbon and hydrogen atoms to be subjected to dehydrogenation.

The dehydrogenation reaction catalyst in accordance with the present embodiment can also be used to promote dehydrogenation reactions other than alkane oxidative coupling reactions. Examples of other dehydrogenation reactions promoted by the dehydrogenation reaction catalyst in accordance with the present embodiment include dehydrogenation reactions of alkanes as illustrated by formula (22) in Fig. 4 and dehydrogenation reactions of alcohols or alcohol derivatives (hereinafter also referred to as "alcohol") as illustrated by formulas (23) and (24). In formulas (22) to (24), A and B each represent a hydrogen atom or an atom or atomic group serving as a substituent with which a hydrogen atom has been replaced as in formulas (12) to (21). Formula (22) represents a reaction that produces an alkene or an alkene derivative from an alkane. Formula (23) shows a reaction that produces an aldehyde from an alcohol, and formula (24) shows a reaction that produces a ketone from an alcohol. Thus, the dehydrogenation reaction catalyst in accordance with the present embodiment can be used as a catalyst that promotes various dehydrogenation reactions.

Even when the dehydrogenation reaction catalyst in accordance with the present embodiment is used for a dehydrogenation reaction other than the methane oxidative coupling reaction shown in Fig. 1, the dehydrogenation reaction catalyst in accordance with the present embodiment can promote a dehydrogenation reaction of a reactant or the like (a reactant provided initially as a raw material or a reactant produced by any of the series of reactions and subjected to the reaction that proceeds successively), such as the reaction from (b) to (c) in Fig. 1. When a reaction such as an alkane oxidative coupling reaction proceeds, an alkane dehydrogenation process that cleaves a stable covalent bond between carbon and hydrogen atoms is generally thought to be a rate-limiting step. Thus, if the dehydrogenation reaction catalyst in accordance with the present embodiment is used to promote the alkane dehydrogenation process described above, the overall dehydrogenation reaction such as an alkane oxidative coupling reaction can be promoted.

When various dehydrogenation reactions involving a process of dehydrogenating a reactant or the like, such as the alkane oxidative coupling reaction described above, are performed, the overall reaction process may be constructed by setting various conditions, such as the pressure during the reaction, the partial pressures of the reactant and the product, the reaction temperature, and the manner of separation and purification of the product, by methods that are well known to or can be easily implemented by those skilled in the art so that the desired compound can be obtained. For example, when a plurality of reactants are used, as shown in formulas (16) to (21), an undesirable reaction may proceed. Specifically, for example, when the reaction of formula (16) proceeds, the reaction of formula (10) or (14), which couples the same reactant with itself, may also proceed. The conditions associated with the reaction as described above may be set as appropriate so that such unintended reactions can be sufficiently inhibited.

Even when the dehydrogenation reaction catalyst in accordance with the present embodiment is used for a dehydrogenation reaction other than a methane oxidative coupling reaction, the activity of promoting a dehydrogenation reaction can be further increased, for example, if the dehydrogenation reaction catalyst has a total conductivity of $1.0 \times 10^{-5}$ S/cm or more and $1.0 \times 10^{-1}$ S/cm or less. In addition, the activity of promoting a dehydrogenation reaction can be further increased if the dehydrogenation reaction catalyst has a proton conductivity of $1.0 \times 10^{-4}$ S/cm or more. In addition, the activity of promoting a dehydrogenation reaction can be further increased if the dehydrogenation reaction catalyst has a proton transport number of 0.01 or more. In addition, the activity of promoting a dehydrogenation reaction can be further increased if x in formula (1), which represents the dehydrogenation reaction catalyst, satisfies inequality (2e) described above. In addition, the activity of promoting a dehydrogenation reaction can be further increased if the dehydrogenation reaction catalyst in accordance with the present embodiment has a perovskite structure represented by the general formula described above, namely, formula (3).

D. Method for Producing Dehydrogenation Reaction Catalyst:

The dehydrogenation reaction catalyst in accordance with the present embodiment can be prepared, for example, by a complex polymerization method. A complex polymerization method is a well-known method that can be used to prepare a composite oxide in which the constituent elements are well mixed. Specifically, this method is performed as follows. Powdered raw materials such as metal nitrates are weighed such that the metal elements in the powdered raw materials are mixed in the ratio corresponding to the composition of the composite oxide to be prepared. The powdered raw materials are dissolved in water, and an oxycarboxylic acid such as citric acid is added thereto to form a metal-oxycarboxylic acid complex. A glycol or the like is then added thereto, and the solution is heated to cause a polymerization reaction to proceed and thereby obtain a polyester polymer gel. This polyester polymer gel is heat-treated (fired) to obtain a composite oxide powder.

The dehydrogenation reaction catalyst in accordance with the present embodiment may also be produced by a method other than the complex polymerization method described above. For example, various methods that can produce composite oxides, such as a solid-phase reaction method, a co-precipitation method, and a sol-gel method, can be used.

When the dehydrogenation reaction catalyst in accordance with the present embodiment is combined with at least one of a metal catalyst and an oxide catalyst into a composite catalyst as described above, the catalysts described above can be combined together using various known methods such as an impregnation method.

E. Supported Catalyst Including Dehydrogenation Reaction Catalyst:

The dehydrogenation reaction catalyst in accordance with the present embodiment may be supported as a catalytic component on a support to form a supported catalyst. In this case, the catalytic component may be a composite catalyst in which the dehydrogenation reaction catalyst in accordance with the present embodiment is combined with at least one of a metal catalyst and an oxide catalyst.

The support can be formed of, for example, a material selected from $CeO_2$, $\gamma$-$Al_2O_3$, zeolite, $SiO_2$, $ZrO_2$, $Na_2WO_4$, $La_2O_3$, $Cs_2SO_4$, $Sm_2O_3$, MgO, SrO, $Y_2O_3$, (La,Sr)$AlO_3$, and $LaAlO_3$. In addition, the support can have a structure such as a foam, a porous body, a porous tube, a honeycomb, or porous particles. The dehydrogenation reaction catalyst or the composite catalyst can be supported on the support by various known methods such as an impregnation method.

F. Apparatus Including Methane Oxidative Coupling Catalyst:

A methane oxidative coupling catalyst that is an example of the dehydrogenation reaction catalyst in accordance with the present embodiment can be used in, for example, an ethylene production apparatus for producing ethylene from methane. The ethylene production apparatus can take various forms. For example, the ethylene production apparatus can be configured as a fixed-bed flow reaction apparatus including a reactor charged with a powdered or granular methane oxidative coupling catalyst.

In this case, the methane oxidative coupling catalyst may be mixed with at least one of a proton conductor and an electron conductor (hereinafter including a hole conductor) as necessary. As the proton conductor, various conventionally known proton conductors can be used. Specifically, for example, perovskite-type composite oxides such as $BaZrO_3$-based oxides, $BaCeO_3$, $SrZrO_3$-based oxides, or $SrCeO_3$-based oxides can be used. As the electron conductor, for example, an electron conductor selected from an oxide electron conductor having a perovskite structure, an oxide electron conductor having a spinel-type crystal structure, and a metal material such as a noble metal can be used. As the oxide electron conductor having a perovskite structure, for example, an LSM-based oxide having Sr added to La sites in a $LaMnO_3$-based compound or a composite oxide such as $SrTiO_3$ can be used.

In addition, the methane oxidative coupling catalyst for the ethylene production apparatus may be supported and held on a support having a shape such as a granular or honeycomb shape. In this case, the support used may include at least one of the proton conductor and electron conductor described above.

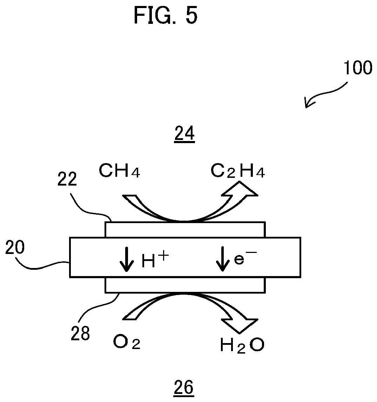

Fig. 5 is an explanatory diagram showing a schematic configuration of an ethylene production apparatus 100 having a different structure as another example of the ethylene production apparatus. The ethylene production apparatus 100 in Fig. 5 includes a ceramic film 20, a first catalyst layer 22 formed one surface of the ceramic film 20, and a second catalyst layer 28 formed on the other surface of the ceramic film 20. The ceramic film 20 is a gas-impermeable dense film formed of a ceramic. The ceramic film 20 has proton conductivity and electron conductivity and is formed of, for example, a mixture of the proton conductor and electron conductor described above. The ceramic film 20 separates the space inside the ethylene production apparatus 100 into two spaces, namely, a first space 24 on the side where the first catalyst layer 22 is formed and a second space 26 on the side where the second catalyst layer 28 is formed.

The first catalyst layer 22 includes the methane oxidative coupling catalyst in accordance with the present embodiment. When the first space 24 is supplied with methane from a methane supply section (not shown) and the second space 26 is supplied with oxygen from an oxygen supply section (not shown), the reactions of formulas (4) and (7) described above proceed over the first catalyst layer 22 to produce ethylene. At this time, the reaction of formula (8) described above also proceeds over the second catalyst layer 28. The catalyst included in the second catalyst layer 28 is preferably an oxide having oxide ion-electron mixed conductivity. For example, $La_{0.6}Sr_{0.4}Co_{0.2}Fe_{0.8}O_3$ can be used.

Various modifications can be made to the ethylene production apparatus 100 in Fig. 5. For example, the ceramic film 20 may have no electron conductivity, and an external circuit that electrically connects together the first catalyst layer 22 and the second catalyst layer 28 may be provided so that the ethylene production apparatus 100 generates electricity during ethylene production. Alternatively, the ceramic film 20 may have oxide ion conductivity instead of proton conductivity. In addition, the second space 26 in the ethylene production apparatus 100 in Fig. 5 may be supplied with an inert gas or carbon dioxide instead of oxygen.

It is sufficient that the methane oxidative coupling catalyst in accordance with the present embodiment be disposed at least at the position where the reaction of formula (4) described above proceeds, irrespective of the type of ethylene production apparatus to which the methane oxidative coupling catalyst is applied. This promotes the reaction of formula (4) so that the ethylene production efficiency can be increased.

EXAMPLES

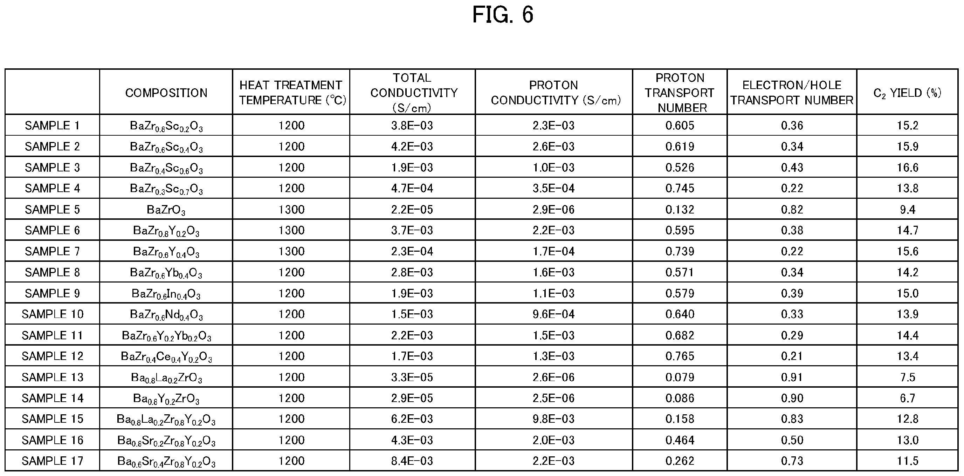

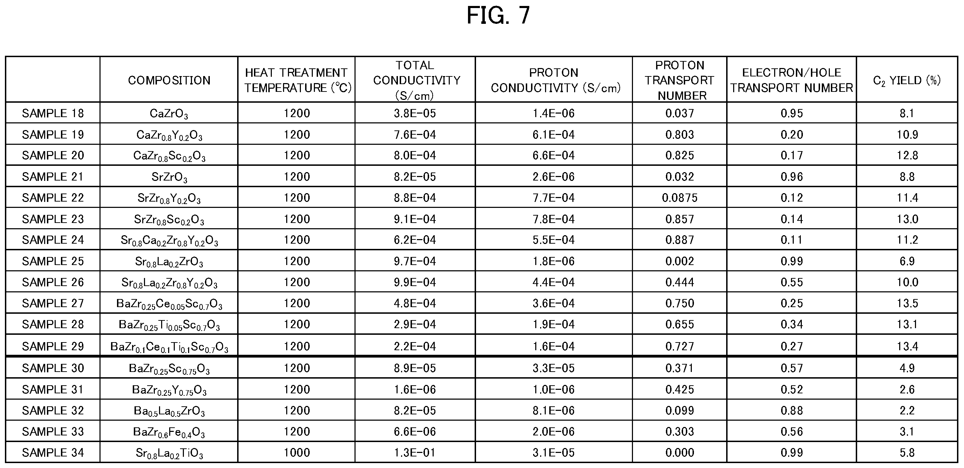

Figs. 6 and 7 are explanatory diagrams showing test results for the performance of 34 methane oxidative coupling catalysts that were prepared, namely, Samples 1 to 34. The configurations, methods of production, and test results for performance of the individual samples will be described below. Of these methane oxidative coupling catalysts, Samples 1 to 29 are composite oxides that satisfy formula (1) and inequalities (2a) to (2d) described above. Samples 30 to 34 are comparative examples.

<Preparation of Samples>

[Sample 1]

A catalyst of Sample 1 ($BaZr_{0.8}Sc_{0.2}O_3$) was prepared by a complex polymerization method. The raw material powders used were barium nitrate (manufactured by FUJIFILM Wako Pure Chemical Corporation), zirconyl nitrate (manufactured by FUJIFILM Wako Pure Chemical Corporation), and scandium nitrate (manufactured by Alfa Aesar). These raw material powders were weighed such that the metal elements were present in the composition ratio of the composition formula $BaZr_{0.8}Sc_{0.2}O_3$. To the raw material powders described above, an aqueous citric acid solution and propylene glycol were added in a ratio of metal element:citric acid:propylene glycol = 1:3:3 (molar ratio), and they were mixed together by stirring at 80°C for 1 hour. Furthermore, this solution was slowly heated to 300°C to obtain a polymer having the metal elements dispersed therein. Furthermore, the resulting polymer was carbonized by heat treatment at 400°C for 2 hours and was pulverized in an agate mortar to obtain a catalyst powder precursor. The resulting catalyst powder precursor was heat-treated at 1,200°C for 6 hours to obtain a powdered methane oxidative coupling catalyst as Sample 1.

Powder XRD analysis confirmed that the powdered methane oxidative coupling catalysts obtained as Sample 1 and Samples 2 to 34 described below had the desired compositions. Specifically, the desired compositions were confirmed as a result of powder XRD analysis based on the fact that a perovskite structure was obtained and that there were no peaks observed for the raw material powders or compounds such as simple oxides (oxides containing only one element besides oxygen) or carbonates derived from the raw material powders. In addition, gas adsorption measurement confirmed that all of the powdered methane oxidative coupling catalysts obtained as Sample 1 and Sample 2 to 34 described below had a specific surface area of 5 $m^2$/g to 10 $m^2$/g.

[Samples 2 to 34]

Powdered methane oxidative coupling catalysts were obtained as Samples 2 to 34 in the same manner as Sample 1 except that the types and weighed amounts of raw material powders for the catalysts were adjusted such that the catalysts had the composition ratios of the composition formulas of Samples 2 to 34 shown in Figs. 6 and 7 and that the catalyst powder precursors were heat-treated at the temperatures shown in Figs. 6 and 7. To add barium, zirconium, scandium, yttrium, ytterbium, indium, neodymium, cerium, lanthanum, strontium, calcium, iron, and titanium as constituent elements, the following raw material powders were used: barium nitrate (manufactured by FUJIFILM Wako Pure Chemical Corporation), zirconyl nitrate (manufactured by FUJIFILM Wako Pure Chemical Corporation), scandium nitrate (manufactured by Alfa Aesar), yttrium nitrate (manufactured by Sigma-Aldrich), ytterbium nitrate (manufactured by Sigma-Aldrich), indium nitrate (manufactured by FUJIFILM Wako Pure Chemical Corporation), neodymium nitrate (manufactured by FUJIFILM Wako Pure Chemical Corporation), cerium nitrate (manufactured by FUJIFILM Wako Pure Chemical Corporation), lanthanum nitrate (manufactured by FUJIFILM Wako Pure Chemical Corporation), strontium nitrate (manufactured by FUJIFILM Wako Pure Chemical Corporation), calcium nitrate (manufactured by FUJIFILM Wako Pure Chemical Corporation), iron nitrate (manufactured by Sigma-Aldrich), and tetra-i-propoxytitanium (manufactured by Kojundo Chemical Lab. Co., Ltd.).

<Measurement of $C_2$ Yield>

Each of Samples 1 to 34 was subjected to a catalytic activity test by weighing 0.1 g of the sample, placing the sample in a fixed-bed flow reaction apparatus, and supplying a gas mixture of methane, oxygen, and nitrogen in a flow ratio of $CH_4:O_2:N_2$ = 3.8:1:4 at 1 atm and 45 $cm^3$/min while heating the sample to 750°C. The gases introduced into and discharged from the apparatus were subjected to compositional analysis by micro gas chromatography (3000A, manufactured by Agilent Technologies). Figs. 6 and 7 show the $C_2$ yield obtained as a result of the compositional analysis. Although $C_2$ hydrocarbons in the gas discharged from the above apparatus included ethylene and ethane, the proportion of ethylene in the resulting $C_2$ hydrocarbons was about 60% to 70% for any sample (no data shown).

<Measurement of Total Conductivity>

Each of Samples 1 to 34 was press-compacted into a rectangular shape and was sintered at 1,600°C for 6 hours. A platinum wire was then wounded around the compact at four positions, and a platinum paste (TR-7905, manufactured by Tanaka Kikinzoku Kogyo K.K.) was applied thereto and baked at 1,000°C for 1 hour to obtain a measurement specimen. Each measurement specimen was placed in an electric tube furnace and was heated while supplying hydrogen humidified with a bubbler, and the total conductivity was measured at 750°C by an AC four-terminal method. Here, relative density measurement by Archimedes' Method for each sintered compact used for total conductivity measurement confirmed that all sintered compacts had a relative density of 96% or more.

<Measurement of Proton Transport Number and Electron/Hole Transport Number>

Each of Samples 1 to 34 was press-compacted into a disk shape and was sintered at 1600°C for 6 hours. A platinum paste was applied in a circular shape to both surfaces of the compact by screen printing and was baked at 1000°C for 1 hour to prepare a measurement specimen. A transport number measurement apparatus included two alumina tubes arranged with the axial directions thereof vertically aligned with each other. The measurement specimen described above was held between the two alumina tubes such that gas sealing properties were ensured between the alumina tubes and the measurement specimen, and the portion including the measurement specimen was placed in an electric furnace and was subjected to transport number measurement. Specifically, the measurement specimen was heated to 750°C using the electric furnace while supplying humidified hydrogen at different concentrations or moisture contents to the spaces inside the two alumina tubes, one space being separated from the other space by the measurement specimen. The electromotive force that occurred between the two surfaces of the measurement specimen was then measured to determine the proton transport number and the electron/hole transport number (the sum of the electron transport number and the hole transport number).

<Calculation of Proton Conductivity>

The proton conductivity was calculated by multiplying the total conductivity measured as described above by the proton transport number.

As shown in Figs. 6 and 7, Samples 1 to 29 exhibited a higher $C_2$ yield than Samples 30 to 34, confirming that a methane oxidative coupling catalyst satisfying formula (1) and inequalities (2a) to (2d) described above exhibits an increased catalytic activity. In addition, Samples 1 to 29, which satisfied formula (1) and inequalities (2a) to (2d) described above, all had a total conductivity of $1.0 \times 10^{-5}$ S/cm or more and $1.0 \times 10^{-1}$ S/cm or less. In addition, of Samples 1 to 29, Samples 1 to 4, 6 to 12, 15 to 17, 19, 20, 22 to 24, and 26 to 29 had a proton conductivity of $1.0 \times 10^{-4}$ S/cm or more. In addition, of Samples 1 to 29, Samples 1 to 24 and 26 to 29 had a proton transport number of 0.01 or more. In addition, of Samples 1 to 29, Samples 1 to 20, 22 to 24, and 26 to 29 had a sum of the electron transport number and the hole transport number of 0.01 or more and 0.95 or less. In addition, of Samples 1 to 29, Samples 1 to 12, 15 to 17, 19, 20, 23, 24, and 26 to 29 had a proton transport number of 0.10 or more and a sum of the electron transport number and the hole transport number of 0.10 or more.

More specifically, for example, when the element A' (at least one of lanthanum (La) and yttrium (Y)) is present in the A sites of the perovskite structure, it is believed to be desirable to satisfy "x ≤ 0.4" (inequality (2a)) or "x ≤ 0.2" (inequality (2e)), for example, as indicated by a comparison between Sample 13 and Sample 32. In particular, it was confirmed that the catalytic activity tends to increase when "x = 0" is satisfied, for example, as indicated by comparisons between Sample 5 and Samples 13 and 14 and between Sample 21 and Sample 25. Furthermore, it was confirmed that the catalytic activity tends to increase when the element A in formula (1), which is an alkaline earth metal, is barium (Ba), for example, as indicated by comparisons between Sample 5 and Samples 18 and 21, between Sample 1 and Samples 20 and 23, and between Sample 6 and Samples 19 and 22. Thus, it was confirmed that it is desirable that the methane oxidative coupling catalyst have a perovskite structure represented by the general formula described above, namely, formula (3).

In addition, it is believed to be desirable to satisfy "0 ≤ z ≤ 0.7" (inequality (2b)), for example, as indicated by comparisons between Samples 1 to 5 and Sample 30 and between Samples 5 to 7 and Sample 31. In addition, it was confirmed that the catalytic activity tends to increase when "0 < z", particularly "0.2 ≤ z", is satisfied, for example, as indicated by comparisons between Samples 1 to 4 and Sample 5 and between Samples 6 and 7 and Sample 5.

The present disclosure is not limited to the above-described embodiments and the like, but can be implemented in various configurations without departing from the spirit thereof. For example, the technical features of the embodiments corresponding to the technical features of the individual aspects described in the Summary of Invention section can be replaced or combined as appropriate to achieve some or all of the objects described above or to achieve some or all of the advantageous effects described above. In addition, these technical features can be deleted as appropriate unless they are described as essential in the present specification.

The present disclosure can also be implemented in the following embodiments:

[Application Example 1]

A dehydrogenation reaction catalyst having a perovskite structure represented by the general formula $(A_{1-x}A'_x)(Zr_{1-y-z}B_yB'_z)O_3$ (where A is at least one element selected from alkaline earth metals, A' is at least one of lanthanum (La) and yttrium (Y), B is at least one of titanium (Ti) and cerium (Ce), and B' is at least one element selected from yttrium (Y), scandium (Sc), ytterbium (Yb), aluminum (Al), indium (In), and neodymium (Nd)), wherein x, Y, and z satisfy:

$$0 \le x \le 0.4,$$

$$0.3 \le (1 - z) \le 1,$$

$$0 \le y,$$

and

$$0 < (1 - y - z).$$

[Application Example 2]

The dehydrogenation reaction catalyst in accordance with application example 1, wherein the dehydrogenation reaction catalyst is an alkane oxidative coupling catalyst that promotes an alkane oxidative coupling reaction that couples a reactant including at least one of an alkane and an alkane derivative.

[Application Example 3]

The dehydrogenation reaction catalyst in accordance with application example 2, wherein the dehydrogenation reaction catalyst is a methane oxidative coupling catalyst.

[Application Example 4]

The dehydrogenation reaction catalyst in accordance with any one of application examples 1 to 3, wherein the dehydrogenation reaction catalyst has a total conductivity of $1.0 \times 10^{-5}$ S/cm or more and $1.0 \times 10^{-1}$ S/cm or less.

[Application Example 5]

The dehydrogenation reaction catalyst in accordance with any one of application examples 1 to 4, wherein the dehydrogenation reaction catalyst has a proton conductivity of $1.0 \times 10^{-4}$ S/cm or more.

[Application Example 6]

The dehydrogenation reaction catalyst in accordance with any one of application examples 1 to 5, wherein the dehydrogenation reaction catalyst has a proton transport number of 0.01 or more.

[Application Example 7]

The dehydrogenation reaction catalyst in accordance with any one of application examples 1 to 6, wherein x satisfies:

$$0 \leq x \leq 0.2.$$

[Application Example 8]

The dehydrogenation reaction catalyst in accordance with any one of application examples 1 to 7, wherein the dehydrogenation reaction catalyst has a sum of an electron transport number and a hole transport number of 0.01 or more and 0.95 or less.

[Application Example 9]

The dehydrogenation reaction catalyst in accordance with any one of application examples 1 to 8, wherein the dehydrogenation reaction catalyst has a proton transport number of 0.10 or more and a sum of an electron transport number and a hole transport number of 0.10 or more.

[Application Example 10]

The dehydrogenation reaction catalyst in accordance with any one of application examples 1 to 9, wherein the dehydrogenation reaction catalyst has a perovskite structure represented by the general formula $BaZr_{1-z}B'_zO_3$ (where B' is at least one element selected from yttrium (Y), scandium (Sc), ytterbium (Yb), indium (In), and neodymium (Nd)).

[Application Example 11]

A composite catalyst comprising the dehydrogenation reaction catalyst in accordance with any one of application examples 1 to 10 and at least one of a metal catalyst and an oxide catalyst.

[Application Example 12]

A supported catalyst comprising a catalytic component supported on a support, wherein the catalytic component includes the dehydrogenation reaction catalyst in accordance with any one of application examples 1 to 10.

[Application Example 13]

A supported catalyst comprising a catalytic component supported on a support, wherein the catalytic component includes the composite catalyst in accordance with claim 11.

Reference Signs List

| 10 | methane oxidative coupling catalyst |
| 20 | ceramic film |
| 22 | first catalyst layer |
| 24 | first space |
| 26 | second space |
| 28 | second catalyst layer |
| 100 | ethylene production apparatus |

**Claims**

1.  A dehydrogenation reaction catalyst having a perovskite structure represented by the general formula $(A_{1-x}A'_x)(Zr_{1-y-z}B_yB'_z)O_3$ (where A is at least one element selected from alkaline earth metals, A' is at least one of lanthanum (La) and yttrium (Y), B is at least one of titanium (Ti) and cerium (Ce), and B' is at least one element selected from yttrium (Y), scandium (Sc), ytterbium (Yb), aluminum (Al), indium (In), and neodymium (Nd)), wherein x, Y, and z satisfy:

$$0 \leq x \leq 0.4,$$

$$0.3 \leq (1 - z) \leq 1,$$

$$0 \leq y,$$

and

$$0 < (1 - y - z).$$

2.  The dehydrogenation reaction catalyst in accordance with claim 1, wherein the dehydrogenation reaction catalyst is an alkane oxidative coupling catalyst that promotes an alkane oxidative coupling reaction that couples a reactant including at least one of an alkane and an alkane derivative.

3.  The dehydrogenation reaction catalyst in accordance with claim 2, wherein the dehydrogenation reaction catalyst is a methane oxidative coupling catalyst.

4.  The dehydrogenation reaction catalyst in accordance with claim 3, wherein

the dehydrogenation reaction catalyst has a total conductivity of $1.0 \times 10^{-5}$ S/cm or more and $1.0 \times 10^{-1}$ S/cm or less.

5. The dehydrogenation reaction catalyst in accordance with claim 3 or 4, wherein
   the dehydrogenation reaction catalyst has a proton conductivity of $1.0 \times 10^{-4}$ S/cm or more.

6. The dehydrogenation reaction catalyst in accordance with claim 3 or 4, wherein
   the dehydrogenation reaction catalyst has a proton transport number of 0.01 or more.

7. The dehydrogenation reaction catalyst in accordance with claim 3 or 4, wherein
   x satisfies:

$$0 \leq x \leq 0.2.$$

8. The dehydrogenation reaction catalyst in accordance with claim 3 or 4, wherein
   the dehydrogenation reaction catalyst has a sum of an electron transport number and a hole transport number of 0.01 or more and 0.95 or less.

9. The dehydrogenation reaction catalyst in accordance with claim 3 or 4, wherein
   the dehydrogenation reaction catalyst has a proton transport number of 0.10 or more and a sum of an electron transport number and a hole transport number of 0.10 or more.

10. The dehydrogenation reaction catalyst in accordance with claim 3 or 4, wherein
    the dehydrogenation reaction catalyst has a perovskite structure represented by the general formula $BaZr_{1-z}B'_zO_3$ (where B' is at least one element selected from yttrium (Y), scandium (Sc), ytterbium (Yb), indium (In), and neodymium (Nd)).

11. A composite catalyst comprising the methane oxidative coupling catalyst in accordance with claim 3 or 4 and at least one of a metal catalyst and an oxide catalyst.

12. A supported catalyst comprising a catalytic component supported on a support, wherein
    the catalytic component includes the methane oxidative coupling catalyst in accordance with claim 3 or 4.

13. A supported catalyst comprising a catalytic component supported on a support, wherein
    the catalytic component includes the composite catalyst in accordance with claim 11.

# FIG. 1

(a)

$$H-\underset{\underset{H}{|}}{\overset{\overset{H}{|}}{C}}-H \quad + \quad O_2$$

⇩

(b)

10

⇩

(c)

10

⇩

(d)

## FIG. 2

10

OXIDATION SITE
$CH_4 \rightarrow \cdot CH_3 + H^+ + e^- \quad \cdots (4)$

REDUCTION SITE
$1/2O_2 + 2H^+ + 2e^- \rightarrow H_2O \quad \cdots (6)$

$CH_4$

$H^+$

$e^-$

$C_2H_4$

$H^+$

$e^-$

$O_2$

$H_2O$

NEAR CATALYST SURFACE
(GAS-PHASE REACTION)
$2 \cdot CH_3 + 1/2O_2 \rightarrow C_2H_4 + H_2O \quad \cdots (5)$

FIG. 3

10

OXIDATION SITE
$CH_4 \rightarrow \cdot CH_3 + H^+ + e^- \quad \cdots (4)$

REDUCTION SITE
$O_2 + 4H^+ + 4e^- \rightarrow 2H_2O \quad \cdots (8)$

$CH_4$

$O_2$

$H^+$

$e^-$

$H^+$

$e^-$

$C_2H_4$

$H_2O$

NEAR CATALYST SURFACE
(GAS-PHASE REACTION)
$2 \cdot CH_3 \rightarrow C_2H_6 \rightarrow C_2H_4 + 2H^+ + 2e^- \cdots (7)$

## FIG. 4

$$CH_4 + CH_4 \rightarrow H_2C{=}CH_2 \quad \cdots \quad (10)$$

$$CH_4 + CH_4 \rightarrow {-}\!\!\left[CH_2{-}CH_2\right]_{\!n} \quad \cdots \quad (11)$$

$$BACH_2 + BACH_2 \rightarrow BAC{=}CAB \quad \cdots \quad (12)$$

$$BACH_2 + BACH_2 \rightarrow {-}\!\!\left[CAB{-}CAB\right]_{\!n} \quad \cdots \quad (13)$$

$$ACH_3 + ACH_3 \rightarrow HAC{=}CAH \quad \cdots \quad (14)$$

$$ACH_3 + ACH_3 \rightarrow {-}\!\!\left[CAH{-}CAH\right]_{\!n} \quad \cdots \quad (15)$$

$$CH_4 + CAH_3 \rightarrow H_2C{=}CAH \quad \cdots \quad (16)$$

$$CH_4 + CAH_3 \rightarrow {-}\!\!\left[CH_2{-}CAH\right]_{\!n} \quad \cdots \quad (17)$$

$$CAH_3 + CA_2H_2 \rightarrow HAC{=}CA_2 \quad \cdots \quad (18)$$

$$CAH_3 + CA_2H_2 \rightarrow {-}\!\!\left[CAH{-}CA_2\right]_{\!n} \quad \cdots \quad (19)$$

$$CH_4 + CABH_2 \rightarrow H_2C{=}CAB \quad \cdots \quad (20)$$

$$CH_4 + CABH_2 \rightarrow {-}\!\!\left[CH_2{-}CAB\right]_{\!n} \quad \cdots \quad (21)$$

$$CH_2ACH_2B \rightarrow HAC{=}CBH \quad \cdots \quad (22)$$

$$ACH_2OH \rightarrow ACH{=}O \quad \cdots \quad (23)$$

$$ABCHOH \rightarrow ABC{=}O \quad \cdots \quad (24)$$

# FIG. 5

## FIG. 6

| | COMPOSITION | HEAT TREATMENT TEMPERATURE (℃) | TOTAL CONDUCTIVITY (S/cm) | PROTON CONDUCTIVITY (S/cm) | PROTON TRANSPORT NUMBER | ELECTRON/HOLE TRANSPORT NUMBER | $C_2$ YIELD (%) |
|---|---|---|---|---|---|---|---|
| SAMPLE 1 | $BaZr_{0.8}Sc_{0.2}O_3$ | 1200 | 3.8E-03 | 2.3E-03 | 0.605 | 0.36 | 15.2 |
| SAMPLE 2 | $BaZr_{0.6}Sc_{0.4}O_3$ | 1200 | 4.2E-03 | 2.6E-03 | 0.619 | 0.34 | 15.9 |
| SAMPLE 3 | $BaZr_{0.4}Sc_{0.6}O_3$ | 1200 | 1.9E-03 | 1.0E-03 | 0.526 | 0.43 | 16.6 |
| SAMPLE 4 | $BaZr_{0.3}Sc_{0.7}O_3$ | 1200 | 4.7E-04 | 3.5E-04 | 0.745 | 0.22 | 13.8 |
| SAMPLE 5 | $BaZrO_3$ | 1300 | 2.2E-05 | 2.9E-06 | 0.132 | 0.82 | 9.4 |
| SAMPLE 6 | $BaZr_{0.8}Y_{0.2}O_3$ | 1300 | 3.7E-03 | 2.2E-03 | 0.595 | 0.38 | 14.7 |
| SAMPLE 7 | $BaZr_{0.6}Y_{0.4}O_3$ | 1300 | 2.3E-04 | 1.7E-04 | 0.739 | 0.22 | 15.6 |
| SAMPLE 8 | $BaZr_{0.6}Yb_{0.4}O_3$ | 1200 | 2.8E-03 | 1.6E-03 | 0.571 | 0.34 | 14.2 |
| SAMPLE 9 | $BaZr_{0.6}In_{0.4}O_3$ | 1200 | 1.9E-03 | 1.1E-03 | 0.579 | 0.39 | 15.0 |
| SAMPLE 10 | $BaZr_{0.6}Nd_{0.4}O_3$ | 1200 | 1.5E-03 | 9.6E-04 | 0.640 | 0.33 | 13.9 |
| SAMPLE 11 | $BaZr_{0.6}Y_{0.2}Yb_{0.2}O_3$ | 1200 | 2.2E-03 | 1.5E-03 | 0.682 | 0.29 | 14.4 |
| SAMPLE 12 | $BaZr_{0.4}Ce_{0.4}Y_{0.2}O_3$ | 1200 | 1.7E-03 | 1.3E-03 | 0.765 | 0.21 | 13.4 |
| SAMPLE 13 | $Ba_{0.8}La_{0.2}ZrO_3$ | 1200 | 3.3E-05 | 2.6E-06 | 0.079 | 0.91 | 7.5 |
| SAMPLE 14 | $Ba_{0.8}Y_{0.2}ZrO_3$ | 1200 | 2.9E-05 | 2.5E-06 | 0.086 | 0.90 | 6.7 |
| SAMPLE 15 | $Ba_{0.8}La_{0.2}Zr_{0.8}Y_{0.2}O_3$ | 1200 | 6.2E-03 | 9.8E-03 | 0.158 | 0.83 | 12.8 |
| SAMPLE 16 | $Ba_{0.8}Sr_{0.2}Zr_{0.8}Y_{0.2}O_3$ | 1200 | 4.3E-03 | 2.0E-03 | 0.464 | 0.50 | 13.0 |
| SAMPLE 17 | $Ba_{0.6}Sr_{0.4}Zr_{0.8}Y_{0.2}O_3$ | 1200 | 8.4E-03 | 2.2E-03 | 0.262 | 0.73 | 11.5 |

# FIG. 7

| | COMPOSITION | HEAT TREATMENT TEMPERATURE (°C) | TOTAL CONDUCTIVITY (S/cm) | PROTON CONDUCTIVITY (S/cm) | PROTON TRANSPORT NUMBER | ELECTRON/HOLE TRANSPORT NUMBER | $C_2$ YIELD (%) |
|---|---|---|---|---|---|---|---|
| SAMPLE 18 | $CaZrO_3$ | 1200 | 3.8E-05 | 1.4E-06 | 0.037 | 0.95 | 8.1 |
| SAMPLE 19 | $CaZr_{0.8}Y_{0.2}O_3$ | 1200 | 7.6E-04 | 6.1E-04 | 0.803 | 0.20 | 10.9 |
| SAMPLE 20 | $CaZr_{0.8}Sc_{0.2}O_3$ | 1200 | 8.0E-04 | 6.6E-04 | 0.825 | 0.17 | 12.8 |
| SAMPLE 21 | $SrZrO_3$ | 1200 | 8.2E-05 | 2.6E-06 | 0.032 | 0.96 | 8.8 |
| SAMPLE 22 | $SrZr_{0.8}Y_{0.2}O_3$ | 1200 | 8.8E-04 | 7.7E-04 | 0.0875 | 0.12 | 11.4 |
| SAMPLE 23 | $SrZr_{0.8}Sc_{0.2}O_3$ | 1200 | 9.1E-04 | 7.8E-04 | 0.857 | 0.14 | 13.0 |
| SAMPLE 24 | $Sr_{0.8}Ca_{0.2}Zr_{0.8}Y_{0.2}O_3$ | 1200 | 6.2E-04 | 5.5E-04 | 0.887 | 0.11 | 11.2 |
| SAMPLE 25 | $Sr_{0.8}La_{0.2}ZrO_3$ | 1200 | 9.7E-04 | 1.8E-06 | 0.002 | 0.99 | 6.9 |
| SAMPLE 26 | $Sr_{0.8}La_{0.2}Zr_{0.8}Y_{0.2}O_3$ | 1200 | 9.9E-04 | 4.4E-04 | 0.444 | 0.55 | 10.0 |
| SAMPLE 27 | $BaZr_{0.25}Ce_{0.05}Sc_{0.7}O_3$ | 1200 | 4.8E-04 | 3.6E-04 | 0.750 | 0.25 | 13.5 |
| SAMPLE 28 | $BaZr_{0.25}Ti_{0.05}Sc_{0.7}O_3$ | 1200 | 2.9E-04 | 1.9E-04 | 0.655 | 0.34 | 13.1 |
| SAMPLE 29 | $BaZr_{0.1}Ce_{0.1}Ti_{0.1}Sc_{0.7}O_3$ | 1200 | 2.2E-04 | 1.6E-04 | 0.727 | 0.27 | 13.4 |
| SAMPLE 30 | $BaZr_{0.25}Sc_{0.75}O_3$ | 1200 | 8.9E-05 | 3.3E-05 | 0.371 | 0.57 | 4.9 |
| SAMPLE 31 | $BaZr_{0.25}Y_{0.75}O_3$ | 1200 | 1.6E-06 | 1.0E-06 | 0.425 | 0.52 | 2.6 |
| SAMPLE 32 | $Ba_{0.5}La_{0.5}ZrO_3$ | 1200 | 8.2E-05 | 8.1E-06 | 0.099 | 0.88 | 2.2 |
| SAMPLE 33 | $BaZr_{0.6}Fe_{0.4}O_3$ | 1200 | 6.6E-06 | 2.0E-06 | 0.303 | 0.56 | 3.1 |
| SAMPLE 34 | $Sr_{0.8}La_{0.2}TiO_3$ | 1000 | 1.3E-01 | 3.1E-05 | 0.000 | 0.99 | 5.8 |

| | INTERNATIONAL SEARCH REPORT | International application No. |
|---|---|---|
| | | **PCT/JP2022/024126** |

**A.    CLASSIFICATION OF SUBJECT MATTER**

*B01J 23/10*(2006.01)i; *B01J 23/02*(2006.01)i; *B01J 23/08*(2006.01)i; *B01J 23/78*(2006.01)i; *C07B 61/00*(2006.01)i; *C07C 2/84*(2006.01)i; *C07C 11/04*(2006.01)i

FI:    B01J23/10 Z; B01J23/02 Z; B01J23/08 Z; B01J23/78 Z; C07B61/00 300; C07C2/84; C07C11/04

According to International Patent Classification (IPC) or to both national classification and IPC

**B.    FIELDS SEARCHED**

Minimum documentation searched (classification system followed by classification symbols)

B01J23/10; B01J23/02; B01J23/08; B01J23/78; C07B61/00; C07C2/84; C07C11/04

Documentation searched other than minimum documentation to the extent that such documents are included in the fields searched

Published examined utility model applications of Japan 1922-1996
Published unexamined utility model applications of Japan 1971-2022
Registered utility model specifications of Japan 1996-2022
Published registered utility model applications of Japan 1994-2022

Electronic data base consulted during the international search (name of data base and, where practicable, search terms used)

**C.    DOCUMENTS CONSIDERED TO BE RELEVANT**

| Category* | Citation of document, with indication, where appropriate, of the relevant passages | Relevant to claim No. |
|---|---|---|
| X | JP 03-262535 A (NIPPON SHOKUBAI CO., LTD.) 22 November 1991 (1991-11-22) claims 1-3, page 3, lower left column, line 11 to page 4, upper right column, line 1 from the bottom, table 2 | 1-3, 6-10 |
| Y | | 11-13 |
| A | | 4-5 |
| Y | JP 04-501871 A (UNION CARBIDE CHEMICALS AND PLASTICS CO., INC.) 02 April 1992 (1992-04-02) claim 1, table 2 | 11-13 |
| A | | 1-10 |
| Y | JP 2008-522811 A (HRD CORP.) 03 July 2008 (2008-07-03) claims 1-32 | 12 |
| A | | 1-11, 13 |
| A | WO 2018/085820 A1 (SABIC GLOBAL TECHNOLOGIES, B.V.) 11 May 2018 (2018-05-11) entire text, all drawings | 1-13 |

☑ Further documents are listed in the continuation of Box C.    ☑ See patent family annex.

| | | | |
|---|---|---|---|
| * | Special categories of cited documents: | "T" | later document published after the international filing date or priority date and not in conflict with the application but cited to understand the principle or theory underlying the invention |
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| **03 August 2022** | **16 August 2022** |

| Name and mailing address of the ISA/JP | Authorized officer |
|---|---|
| **Japan Patent Office (ISA/JP)** **3-4-3 Kasumigaseki, Chiyoda-ku, Tokyo 100-8915** **Japan** | |
| | Telephone No. |

Form PCT/ISA/210 (second sheet) (January 2015)

**INTERNATIONAL SEARCH REPORT**

| International application No. |
| --- |
| **PCT/JP2022/024126** |

### C. DOCUMENTS CONSIDERED TO BE RELEVANT

| Category* | Citation of document, with indication, where appropriate, of the relevant passages | Relevant to claim No. |
| --- | --- | --- |
| A | CN 112547039 A (SHANXI INSTITUTE OF COAL CHEMISTRY, CHINESE ACADEMY OF SCIENCES) 26 March 2021 (2021-03-26)<br>entire text, all drawings | 1-13 |

Form PCT/ISA/210 (second sheet) (January 2015)

**INTERNATIONAL SEARCH REPORT**
Information on patent family members

International application No.

**PCT/JP2022/024126**

| Patent document cited in search report | | | Publication date (day/month/year) | Patent family member(s) | | | Publication date (day/month/year) |
|---|---|---|---|---|---|---|---|
| JP | 03-262535 | A | 22 November 1991 | (Family: none) | | | |
| JP | 04-501871 | A | 02 April 1992 | WO | 91/04237 | A1 | |
| | | | | claim 1, table 2 | | | |
| | | | | EP | 418975 | A1 | |
| | | | | CN | 1050864 | A | |
| JP | 2008-522811 | A | 03 July 2008 | US | 2006/0135838 | A1 | |
| | | | | claims 1-40 | | | |
| | | | | WO | 2006/063230 | A1 | |
| | | | | EP | 1843841 | A1 | |
| WO | 2018/085820 | A1 | 11 May 2018 | US | 2019/0329223 | A1 | |
| | | | | CN | 109890501 | A | |
| CN | 112547039 | A | 26 March 2021 | (Family: none) | | | |

Form PCT/ISA/210 (patent family annex) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **BRITTANY LANCASTER FARRELL et al.** *ACS Catal.*, 2016, vol. 6, 4340-4346